# EUROPÄISCHE PATENTANMELDUNG

(11) **EP 0 745 356 A2**
(43) Veröffentlichungstag der Anmeldung: **04.12.1996**
(21) Anmeldenummer: 96113786.6
(22) Anmeldetag: 10.05.1994
(51) Int. Cl.: A61B 17/74

(54) **Osteosynthese-Hilfsmittel zur Versorgung subtrochanterer, pertrochanterer und Schenkelhalsfrakturen**

(30) Priorität: 01.06.1993 DE 4318150
(62) Teilanmeldung aus: 94917609.3
(71) Anmelder: ENDOCARE AG, CH-6343 Rotkreuz (CH)
(72) Erfinder: Friedl, Wilhelm, 69214 Eppelheim (DE)
(74) Vertreter: Popp, Eugen, Dr.

(57) **Zusammenfassung**

Die Erfindung betrifft ein Osteosynthese-Hilfsmittel (10) zur Versorgung subtrochanterer, pertrochanterer und Schenkelhalsfrakturen mit einem von proximal in den Markraum eines Femur (11) einführbaren Verriegelungsnagel (12), der einen distalen Bereich (15) sowie einen proximalen Abschnitt (14) mit einer schrägen Durchgangsöffnung (16) aufweist. Durch die schräge Durchgangsöffnung (16) hindurch ist von lateral in den Schenkelhals (17) und Femurkopf (18) ein Schenkelhalsteil (19) einführbar. Der Schenkelhalsteil (19) weist an seinem äußeren, dem Fermurkopf (18) abgewandten Ende einen radial vorspringenden Absatz, insbesondere einen sich über den Umfang erstreckenden Kragen (33) auf, der die laterale Einführung des Schenkelhalsteils (19) in die schräge Durchgangsöffnung (16) des Verriegelungsnagels (12) begrenzt.

## Beschreibung

Die Erfindung betrifft ein Osteosynthese-Hilfsmittel zur Versorgung subtrochanterer, pertrochanterer und Schenkelhalsfrakturen gemäß dem Oberbegriff des Patentanspruches 1.

Ein derartiges Osteosynthese-Hilfsmittel ist z.B. aus der EP-A-521 600 bekannt. Bei dem bekannten Osteosynthese-Hilfsmittel dient der Verriegelungsnagel zur Führung und Halterung eines zugeordneten Schenkelhalsteils in Form einer massiven, eine zentrale Durchgangsbohrung aufweisenden Schaftschraube. Zu diesem Zweck weist der Verriegelungsnagel im proximalen Bereich eine schräge Durchgangsöffnung auf, durch die die Schaftschraube bzw. Schenkelhalsschraube hindurchführbar ist. Am femurkopfseitigen Ende der Schenkelhalsschraube ist ein selbstschneidendes Gewinde ausgebildet, mit dem die Verankerung im Femurkopf erfolgt. Die Schenkelhalsschraube ist in der Durchgangsöffnung des Verriegelungsnagels innerhalb einer Hülse geführt. Diese Hülse läßt sich nach Einschlagen des Verriegelungsnagels seitlich in die Durchgangsöffnung einbringen und dort verriegeln. Anschließend wird die Schenkelhalsschraube durch die erwähnte Hülse in der Durchgangsöffnung hindurchgeführt und in den Femurkopf eingeschraubt.

Nachteilig ist hierbei, daß die Einschraubtiefe der Schenkelhalsschraube nicht begrenzt werden kann, so daß ein nicht unbeachtliches Risiko besteht, daß die Schenkelhalsschraube zu weit in den Femurkopf eingeschraubt wird. Von weiterem Nachteil ist ferner, daß die Hülse während der Operation als separates Teil zusätzlich zur Schenkelhalsschraube in den Verriegelungsnagel eingeführt und dort befestigt werden muß, was zum einen die Operation verkompliziert und zum anderen die Operationszeit verlängert.

Des weiteren ist in der US 3,561,437 B2 eine Vorrichtung zur Fixierung von Schenkelhalsbrüchen beschrieben. Die Vorrichtung besteht aus einer Leiste, die mittels Schrauben am oberen Bereich des Femurs befestigt wird, sowie aus einer Hülse, die am proximalen Ende der Leiste angeordnet ist und eine schräge Durchgangsöffnung bildet. Die Hülse weist einen kleeblattförmigen Querschnitt auf und ist nach oben bzw. proximal offen. Anstelle der Leiste kann gemäß einer weiteren Ausführungsform ein Stift mit einem ebenfalls offenen kleeblattförmigen Querschnitt vorgesehen sein, der in den intramedulären Kanal des Femurschaftes eingeführt wird. Durch die Hülse wird ein Schenkelhalsnagel vom Smith-Petersen-Typ in den Femurkopf eingeschlagen. Um die Einführtiefe des Schenkelhalsnagels in den Fermurkopf zu begrenzen, ist der Durchmesser am Kopf des Schenkelhalsnagels verglichen mit dem Durchmesser des Schenkelhalsnagelkörpers geringfügig vergrößert, wobei der Durchmesser ausgehend vom Schenkelhalskörper kontinuierlich zunimmt.

Durch die kontinuierliche und insgesamt geringfügige Zunahme des Durchmessers am Kopf des Schenkelhalsnagels verbleibt jedoch, nicht zuletzt auch aufgrund der Halterung desselben in der nach proximal offenen Hülse, ausreichend Spiel in Bezug auf die Einführtiefe, so daß auch hier die Gefahr besteht, daß der Schenkelhalsnagel zu tief in den Fermurkopf eingeschlagen wird. Des weiteren besteht die Gefahr, daß eine derartige Vorrichtung den im Fermurbereich auftretenden hohen Kräften, insbesondere den hohen Druckbelastungen nicht standhält. Hierbei kann es leicht zu Verformungen oder Verbiegungen der Hülse kommen, so daß ein nicht unbeachtliches Risiko besteht, daß sich die proximale Öffnung der Hülse vergrößert und der Schenkelhalsnagel letztlich freiliegt bzw. ausbricht.

Aufgabe der vorliegenden Erfindung ist es daher, ein Osteosynthese-Hilfsmittel der bekannten Art so weiterzubilden, daß eine einfach zu handhabende sowie stabile und zugleich schonende Fixierung eines Schenkelhalssteils im Knochen gewährleistet ist.

Diese Aufgabe wird durch die kennzeichnenden Merkmale des Patentanspruches 1 gelöst.

Ein Kerngedanke der vorliegenden Erfindung liegt darin, daß der Schenkelhalsteil, d.h. entweder Schenkelhalsklinge oder herkömmliche Schenkelhalsschraube an seinem bzw. ihrem äußeren, dem Femurkopf abgewandten Ende einen radial vorspringenden Absatz, insbesondere einen sich über den Umfang erstreckenden Kragen aufweist, der die laterale Einführung des Schenkelhalsteils in die schräge Durchgangsöffnung des Verriegelungsnagels begrenzt. Damit kann der Schenkelhalsteil nicht aus Versehen zu weit in die schräge Durchgangsöffnung des Verriegelungsnagels eingeschlagen werden mit der Gefahr, daß bei Belastung der Schenkelhalsteil in Varusposition abkippt. Es hat sich in der Praxis gezeigt, daß bei einem solchem Abkippen des Schenkelhalsteils dieser kaum noch auf normalem Wege entfernbar ist. Des weiteren kann durch die Erfindung verhindert werden, daß der Schenkelhalsteil durch den Femurkopf nach außen dringt.

Vorzugsweise ist gemäß Anspruch 2 der Schenkelhalsteil als Schenkelhalsklinge ausgebildet, wobei der im Femurkopf verankerbare Abschnitt als Doppel-T bzw. I-, T-, Stern-, U- oder dgl. -profil ausgebildet ist, wobei dieses vorzugsweise noch eine zentrale Durchgangsbohrung für einen Führungsdraht umfaßt. Gemäß Anspruch 3 ist das vordere bzw. im Femurkopf liegende Ende bzw. sind die vorderen Kanten des Profilabschnitts der Schenkelhalsklinge als Schneiden ausgebildet. Die vorgenannte Konstruktion der Schenkelhalsklinge zeichnet sich durch ein hohes Flächenträgheitsmoment und damit einen entsprechend hohen Biegewiderstand bei vergleichsweise dünnwandiger Ausbildung aus. Es muß daher beim Einbringen bzw. Einschlagen der Schenkelhalsklinge in den Schenkelhals und Femurkopf nur Spongiosa verdrängt werden. Die Einbringung der Schenkelhalsklinge ist vergleichsweise schonend, und zwar auch bedingt durch die Ausbildung der vorderen Kanten als Schneiden.

Die vorgenannte Profilkonstruktion gewährleistet auch die Rotationsstabilität des Femurkopfes und/oder Schenkelhalses gegenüber der Schenkelhalsklinge.

Sollte im übrigen die Schenkelhalsklinge mit Profil-Abschnitt einen rotationssymestrischen Querschnitt aufweisen, müßte eine zusätzliche Rotationssicherung im Bereich der schrägen Durchgangsöffnung des Verriegelungsnagels vorgesehen sein.

Nachstehend wird eine Ausführungsform eines erfindungsgemäß ausgebildeten Osteosynthese-Hilfsmittel anhand der beigefügten Zeichnungen näher erläutert. Es zeigen:
- Fig. 1: einen erfindungsgemäß ausgebildeten Verriegelungsnagel in Lateralansicht;
- Fig. 2: den Verriegelungsnagel gemäß Fig. 1 teilweise im Längsschnitt, teilweise in Ansicht;
- Fig. 3: eine Draufsicht auf das proximale Ende des Verriegelungsnagels in Richtung der Pfeile A-A in Fig. 2 und in vergrößertem Maßstab;
- Fig. 4: eine dem Verriegelungsnagel gemäß den Fig. 1 - 3 zugeordnete Schenkelhalsklinge in Seitenansicht;
- Fig. 5: das laterale (distale) Ende der Schenkelhalsklinge gemäß Fig. 4 in Draufsicht;
- Fig. 6: das mediale (proximale) Ende der Schenkelhalsklinge gemäß Fig. 4 in Draufsicht und in vergrößertem Maßstab;
- Fig. 7: den Profilabschnitt der Schenkelhalsklinge gemäß Fig. 4 im Schnitt längs Linie A-A in Fig. 4 und in vergrößertem Maßstab; und
- Fig. 8: die Zuordnung zwischen Verriegelungsnagel, Schenkelhalsklinge und zugeordnetem Zielgerät relativ zueinander und relativ zum Femur in schematischer Vorderansicht.

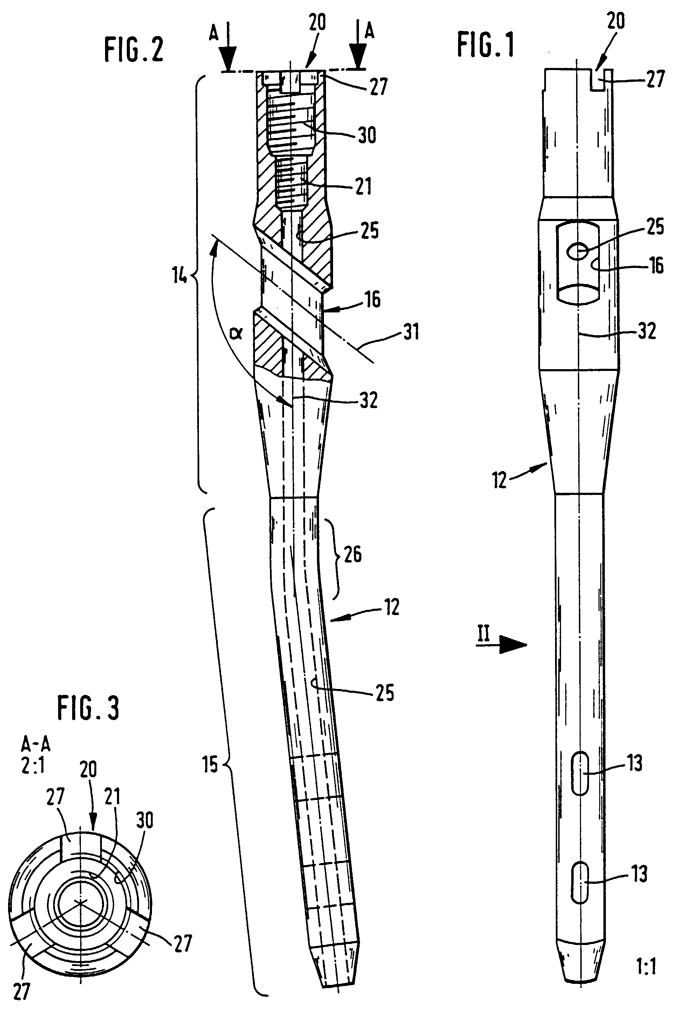

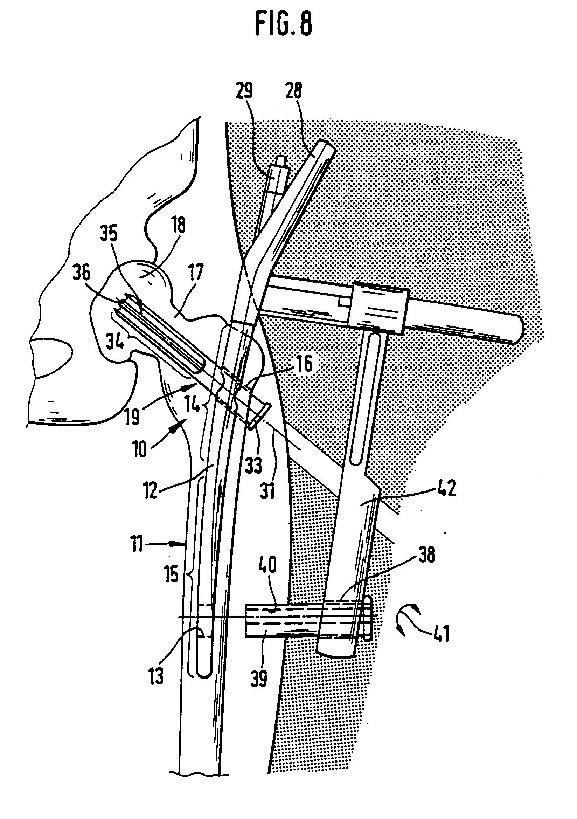

In den Fig. 1 - 8 ist ein Osteosynthese-Hilfsmittel 10 (siehe Fig. 8) zur Versorgung subtrochanterer und pertrochanterer sowie Schenkelhalsfrakturen mit einem von proximal in den Markraum eines Femur 11 einführbaren Verriegelungsnagel 12, der einen distalen Bereich mit zwei Langloch-Querbohrungen 13 für die Aufnahme eines distalen Verriegelungselements, nämlich nicht näher dargestellten Verriegelungsbolzens, sowie einen proximalen Abschnitt 14 mit einer schrägen Durchgangsöffnung 16 aufweist, und mit einem von lateral durch die schräge Durchgangsöffnung 16 in den Schenkelhals 17 und Femurkopf 18 einführbaren Schenkelhalsteil in Form einer Schenkelhalsklinge 19 mit rechteckförmigem Grundquerschnitt dargestellt. Der distale Bereich des Verriegelungsnagels 12 ist mit der Bezugsziffer 15 gekennzeichnet.

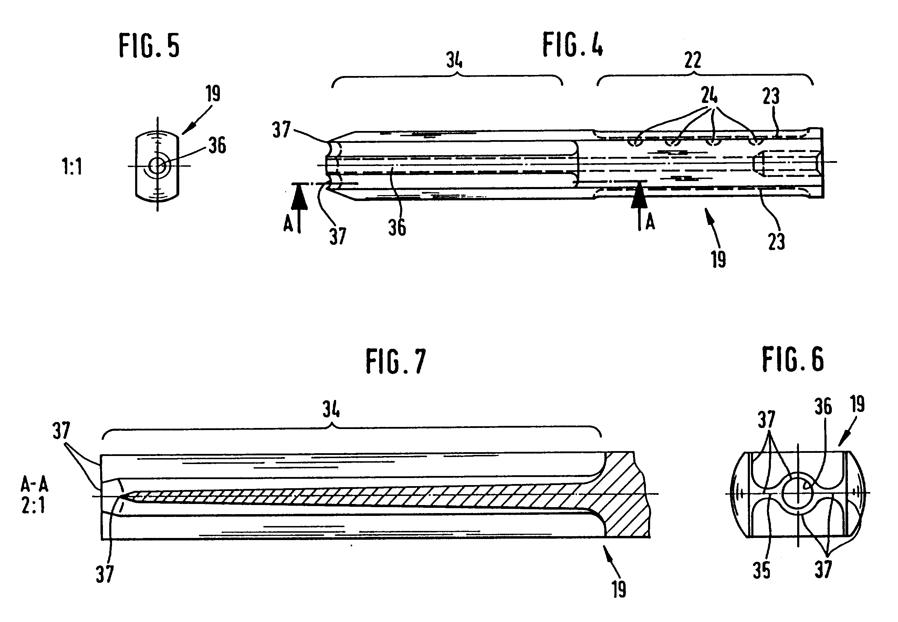

Entsprechend dem Querschnitt der Schenkelhalsklinge 19 ist der Querschnitt der schrägen Durchgangsöffnung 16 ausgebildet, wobei Schenkelhalsklinge 19 und Durchgangsöffnung 16 so relativ zueinander dimensioniert sind, daß die Schenkelhalsklinge 19 innerhalb der Durchgangsöffnung 16 spielfrei gehalten ist. In das proximale Ende 20 des Verriegelungsnagels 12 ist ein nicht näher dargestellter Verriegelungsstift (Madenschraube oder dgl.) einsetzbar, nämlich einschraubbar. Die entsprechende Gewindebohrung ist in Fig. 2 mit der Bezugsziffer 21 gekennzeichnet. Mittels des in die Gewindebohrung 21 einschraubbaren Verriegelungsstiftes kann die axiale Bewegung der Schenkelhalsklinge 19 innerhalb der schrägen Durchgangsöffnung 16 entweder begrenzt oder vollständig blockiert werden. Zu diesem Zweck weist der der Durchgangsöffnung 16 zugeordnete Abschnitt 22 (siehe Fig. 4) der Schenkelhalsklinge 19 zumindest an der proximalen (bei der hier dargestellten Ausführungsform auch an der distalen) Seite eine achsparallele Nut 23 auf, in die das in das proximale Ende 20 des Verriegelungsnagels 12 einsetzbare Verriegelungselement bzw. der vorerwähnte Verriegelungsstift in der beschriebenen Weise eingreift. Die eine Nut 23 kann zusätzlich im axialen Abstand voneinander angeordnete Vertiefungen 24 aufweisen, in die bei Bedarf der Verriegelungsstift eingreifen soll, um eine sichere starre Verbindung zwischen Verriegelungsnagel 12 und Schenkelhalsklinge 19 herstellen zu können. Damit soll bei Bedarf eine Axialbewegung der Schenkel-halsklinge 19 sicher vermieden werden. Je nach Bedarf wird bei der Schenkelhalsklinge 19 gemäß Fig. 4 entweder die Axialnut 23 mit den muldenförmigen Vertiefungen 24 oder die Axialnut 23 ohne die vorgenannten Vertiefungen dem Verriegelungsstift zugeordnet. Die erstgenannte Zuordnung wird insbesondere dann gewählt, wenn die Schenkelhalsklinge 19 innerhalb der schrägen Durchgangsöffnung 16 des Verriegelungsnagels 12 blockiert werden soll. Insofern ist die Ausführungsform gemäß Fig. 4 doppelfunktional.

Wie bereits erwähnt, ist der Schenkelhalsteil bei der hier beschriebenen Ausführungsform als Schenkelhalsklinge 19 mit einem von einem kreis- bzw. rotationssymmetrischen Querschnitt abweichenden Querschnitt, nämlich Rechteck-Querschnitt ausgebildet. Dadurch und durch den entsprechenden Querschnitt der schrägen Durchgangsöffnung 16 im Verriegelungsnagel 12 wird eine dauerhaft drehfeste Plazierung der Schenkelhalsklinge 19 innerhalb der schrägen Durchgangsöffnung 16 erreicht. Die rechteckförmige Durchgangsöffnung 16 ist so ausgebildet, daß die Schenkelhalsklinge 19 hochkant in der Durchgangsöffnung 16 plaziert ist. Die Flachseiten der Schenkelhalsklinge 19 erstrecken sich also parallel zur Längsachse des Verriegelungsnagels 12 bzw. Schenkelhalses. Die beschriebene Konstruktion führt zu einem besonders großen Biegewiderstand der Schenkelhalsklinge mit der Folge, daß keine Gefahr für ein Blockieren der Axialbewegung der Schenkelhalsklinge 19 bedingt durch eine Verbiegung derselben besteht. Bei dynamischer Plazierung der Schenkelhalsklinge 19 innerhalb der Durchgangsöffnung 16 bleibt die Dynamik sicher erhalten.

Wie der Fig. 2 entnommen werden kann, ist der Verriegelungsnagel 12 durchgehend hohl ausgebildet (Durchgangsbohrung 25), so daß er über einen nicht näher dargestellten, jedoch an sich bekannten Führungsspieß in den proximalen Femur einführbar ist. Die Wandstärke des hohlen Verriegelungsnagels 12 beträgt etwa 1,5 bis 2,5 mm, insbesondere etwa 2,0 mm. Im übrigen ist der Verriegelungsnagel aus einem humanverträglichen Material, insbesondere Titan bzw. einer Titanlegierung hergestellt. Das gleiche gilt für die Schenkelhalsklinge 19 sowie alle anderen Teile, wie Schenkelhalsklingen-verriegelungsstift und distale Verriegelungsbolzen.

Wie des weiteren der Fig. 2 und auch der Fig. 8 entnommen werden kann, ist in der durch Verriegelungsnagel 12 und Schenkelhalsklinge 19 definierten Ebene der proximale Abschnitt 14 des Verriegelungsnagels 12 gegenüber dessen distalem Abschnitt 15 um etwa 4° - 8°, insbesondere etwa 6° lateral nach außen gebogen, wobei der Übergang zwischen distalem und proximalem Abschnitt durch einen knickfreien Bogenabschnitt 26 gebildet ist.

Im übrigen weist der Verriegelungsnagel 12 bei der dargestellten Ausführungsform einen rotationssymmetrischen bzw. kreisrunden Querschnitt auf. Grundsätzlich ist auch ein ovaler Querschnitt denkbar, um die Rotationsstabilität des Osteosynthese-Hilfsmittels innerhalb des Femur zu fördern.

Zu dem nicht näher dargestellten Verriegelungsstift für die Schenkelhalsklingen 19 sei noch erwähnt, daß dieser in an sich bekannter Weise versenkt im proximalen Ende 20 des Verriegelungsnagels 12 angeordnet ist.

Um die Festigkeit des Verriegelungsnagels 12 im kritischen Bereich der Durchgangsöffnung 16 zu erhöhen, ist dieser Bereich im Verhältnis zum distalen Bereich 15 des Verriegelungsnagels 12 aufgeweitet. Konkret ist der Außendurchmesser des Verriegelungsnagels 12 im Bereich der Durchgangsöffnung 16 etwa doppelt so groß wie der Durchmesser des distalen Bereichs 15. Damit ist gewährleistet, daß auch im Bereich der Durchgangsöffnung 16 die oben genannte Wandstärke des Verriegelungsnagels 12 erhalten wird. Die Gefahr eines Bruchs des Verriegelungsnagels 12 im Bereich der Durchgangsöffnung 16 wird auf diese Weise ausgeschlossen.

Im übrigen sollen sämtliche Übergänge zwischen distalem Bereich 15 und verbreitertem proximalen Abschnitt 14 knickfrei bzw. sanft gewölbt ausgebildet sein, um Spannungsspitzen in den genannten Übergangsbereichen zu vermeiden.

Entsprechend den Fig. 1 - 3, insbesondere entsprechend Fig. 3 sind am proximalen Ende 20 des Verriegelungsnagels 12 drei gleichmäßig über den Umfang verteilt angeordnete Einschnitte 27 zur formschlüssigen Verbindung mit komplementären Vorsprüngen an einem Zielgerät ausgebildet, so wie es in Fig. 8 mit der Bezugsziffer 28 angedeutet ist. Durch diese formschlüssige Verbindung, die zusätzlich kombiniert wird mit einer kraftschlüssigen Schraubverbindung (Schraubbolzen 29 in Fig. 8 und zugehörige Gewindebohrung 30 im proximalen Ende 20 des Verriegelungsnagels 12 entsprechend Fig. 2), wird eine starre Verbindung zwischen Zielgerät 28 und verriegelungs-nagel 12 erhalten, die erforderlich ist, um die Schenkelhalsklinge 19 sowie distalen Verriegelungsbolzen zielgenau positionieren zu können.

Die Längsachse 31 der schrägen Durchgangsöffnung 16 für die Schenkelhalsklinge 19 schließt mit der Längsachse 32 des proximalen Abschnitts 14 des Verriegelungsnagels 12 einen Winkel a von entweder 125° oder alternativ 135° ein. Mit diesen beiden Winkeln lassen sich alle physiologischen Variationen des Schenkelhals-Fermurschaft-Winkels beherrschen. Eine ausgeprägte Valgisationsstellung um 140° und mehr ist bei dem erfindungsgemäßen System, das nicht von einer knöchernen kortikalen Kraftübertragung abhängig ist, nicht erforderlich. Dementsprechend sind bei Verwendung des Osteosynthese-Hilfsmittels auch nur zwei Zielbögen erforderlich, und zwar ein Zielbogen für 125° und ein weiterer Zielbogen für 135°. Man kommt also mit einer minimalen Anzahl von Instrumenten aus.

Die Schenkelhalsklinge 19 weist entsprechend Fig. 8 an ihrem äußeren, dem Femurkopf 18 abgewandten Ende einen radial vorspringenden Absatz in Form eines sich über den Umfang erstreckenden Kragens 33 auf, der die laterale Einführung der Schenkelhalsklinge 19 in die schräge Durchgangsöffnung 16 des Verriegelungsnagels 12 begrenzt. Dieser radial vorspringende Absatz bzw. Kragen 33 kann auch mit Erfolg bei herkömmlichen Schenkelhalsschrauben ausgeführt sein, um die eingangs genannten Gefahren beim Einschlagen der Schenkelhalsklinge bzw. Schenkelhalsschraube zu vermeiden. Insofern handelt es sich um ein gesondertes und für die Praxis bedeutendes Konstruktionsmerkmal.

Entsprechend den Fig. 4, 6, 7 und 8 ist der im Femurkopf 18 und Schenkelhals 17 verankerbare Abschnitt 34 der Schenkel-halsklinge 19 als Doppel-T bzw. I-Profil 35 mit einer zentralen Durchgangsbohrung 36 für einen nicht näher dargestellten, jedoch an sich bekannten Führungsdraht ausgebildet. Die vorderen im Femurkopf 18 liegenden Kanten des Profilabschnitts 34 sind als meißelartige Schneiden 37 ausgebildet, um das Einschlagen der Schenkelhalsklinge 19 in die Spongiosa des Schenkelhalses und Femurkopfes ohne vorheriges Aufbohren der Spongiosa auf volle Breite der Schenkelhalsklinge zu erleichtern. Wie bereits eingangs erwähnt, wird mit der beschriebenen Ausbildung der Schenkelhalsklinge 19 mit einem Doppel-T bzw. I-Profilabschnitt 34 ein extrem hohes Flächenträgheitsmoment und damit extrem hohe Biegesteifigkeit bei minimaler Querschnittsfläche erreicht, so daß beim Einschlagen der Schenkelhalsklinge in den Schenkelhals und Femurkopf nur wenig Spongiosa verdrängt werden muß. Darüber hinaus begünstigt der Profilabschnitt 34 die Rotationsstabilität des Femurkopfes und Schenkelhalses im Verhältnis zum Femur bei Versorgung eines Schenkelhalsbruches oder einer pertrochanteren Fraktur.

Die beiden distalen Querbohrungen 13 im distalen Bereich des Verriegelungsnagels 12 sind als Langloch-Querbohrungen ausgebildet, durch die hindurch distale Verriegelungselemente, nämlich Verriegelungsbolzen zur Verankerung im Femurknochen durchführbar sind, wobei die Verriegelungsbolzen zur statischen distalen Verriegelung des Verriegelungsnagels 12 am proximalen Ende (in Fig. 1 und 2 oberen Ende) und zur dynamischen distalen Verriegelung des Verriegelungsnagels 12 am distalen Ende (in Fig. 1 und 2 unteren Ende) der Langloch-Querbohrungen 13 eingeführt werden. Bei der dargestellten Ausführungsform weisen die Langloch-Querbohrungen 13 jeweils eine axiale Erstreckung bzw. Länge auf, die etwa dem 2,5-fachen Durchmesser der zugeordneten Verriegelungsbolzen bzw. der Breite der Langloch-Querbohrungen 13 entspricht.

Die distalen Verriegelungsbolzen werden mittels eines Zielgeräts 28 eingebracht. Wie bereits oben ausgeführt, sollen die distalen Verriegelungsbolzen an ihren distalen Enden jeweils ein selbstschneidendes Gewinde mit minimaler Gewindetiefe aufweisen. Das Gewinde soll gerade ausreichen, um die Verriegelungsbolzen im Femurknochen zu halten. Eine Klemm-Verschraubung soll zur Schonung des Knochens im Gegensatz zum Stand der Technik nicht möglich sein.

Der durchschnittliche Durchmesser des erfindungsgemäßen Verriegelungsnagels beträgt etwa 11 bis 14 mm, vorzugsweise etwa 12 mm. Dieser relativ geringe Durchmesser reicht aus, da keine Kraftübertragung vom Verriegelungsnagel auf den Knochen stattfindet, bzw. stattfinden soll. Die Krafteinleitung erfolgt über die Schenkelhalsklinge, den intramedullären Verriegelungsnagel auf die distalen Verriegelungselemente und von dort auf den distalen Fermur. Ein großer rigider Verriegelungsnagel, der einen Knochenkontakt zum Femur herstellen würde, wäre nur von Nachteil. Es bestünde die Gefahr, daß der Femur durch den Nagel gesprengt wird, sowie erhöhte Frakturgefahr am Nagelende.

Anhand der Fig. 8 sei noch auf ein für das beschriebene Osteosynthese-Hilfsmittel geeignetes Zielgerät mit der Bezugsziffer 28 hingewiesen. Dieses ist am proximalen Ende 20 des Verriegelungsnagels 12 unter Ausbildung einer starren Verbindung mit diesem anschließbar. Es weist an einem im montierten Zustand sich etwa parallel zum Verriegelungsnagel 12 und in Richtung zu dessen distalem Ende hin mindestens ein der mindestens einen im distalen Bereich des Verriegelungsnagels 12 ausgebildeten Langloch-Querbohrung 13 zugeordnetes Langloch 38 zur Aufnahme einer komplementären Zielbüchse 39 auf, die an ihrem einen Ende eine Bohrung 40 umfaßt, deren Innendurchmesser geringfügig größer ist als der Außendurchmesser eines durch diese Bohrung hindurchzuführenden distalen Verriegelungsbolzens. Die Zielbüchse 39 ist im Langloch 38 um 180° wendbar, so daß die Bohrung 40 entweder dem proximalen oder dem distalen Ende der zugeordneten Langloch-Querbohrung 13 im distalen Bereich des Verriegelungsnagels 12 zustellbar ist. Die Möglichkeit, die Zielbüchse 39 in der beschriebenen Weise um 180° zu wenden, ist in Fig. 8 mit dem Doppelpfeil 41 angedeutet. Die Bohrung 40 dient zugleich als Bohrhülse für einen Bohrer, mit dem die Aufnahmeöffnungen im Femur für die distalen Verriegelungsbolzen vorgebohrt werden. In Verlängerung der Schenkelhalsklinge 19 weist der vorgenannte Arm 42 einen Durchgang zur Aufnahme einer Bohr- und Zentrierhülse für die Schenkelhalsklinge auf. Diese beiden Hülsen sind in Fig. 8 nicht näher dargestellt, da es sich diesbezüglich um an sich bekannte Konstruktionselemente handelt.

Mit diesem Zielgerät wird ferner die Schenkelhalsklinge eingeführt, wobei der entsprechende Bereich im Schenkelhals und Femurkopf aufgebohrt wird, und zwar mittels eines Stufenbohrers, dessen vorderer Durchmesser etwa 3,5 mm und im übrigen etwa 10 mm beträgt, wobei der Bereich mit einem Durchmesser von 10 mm zum Aufbohren des Aufnahmeraums für den lateralen (distalen) Abschnitt der Schenkelhalsklinge dient. Für den medialen (proximalen) Profil-Abschnitt der Schenkel-halsklinge genügt eine Vorbohrung mit einem Durchmesser von etwa 3,5 mm. Die Aufbohrung erfolgt über einen Zieldraht für die Schenkel-halsklinge.

Im übrigen ist das Zielgerät 28 einstückig ausgebildet, um Relativverschiebungen von einzelnen Bauteilen des Zielgerätes zu vermeiden. Dadurch wird eine hohe Zielgenauigkeit für das Einbringen der Schenkelhalsklinge sowie der distalen Verriegelungsbolzen nach Anschluß am Verriegelungsnagel 12 erreicht.

Werden sämtliche Konstruktionsmerkmale des beschriebenen Systems vereinigt, erhält man im Vergleich zum Stand der Technik ein optimales Osteosynthese-Hilfsmittel, wie erste Versuche des Erfinders bereits gezeigt haben.

Die obigen Ausführungen lassen schließlich noch erkennen, daß man bei Verwendung des erfindungsgemäßen Osteosynthese-Hilfsmittels eine minimale Anzahl von Instrumenten benötigt, nämlich:
- Zielgerät 125°;
- Zielgerät 135°;
- Bohr- und Zentrierhülse für die Schenkelhalsklinge;
- Bohr- und Zielbüchse für die distalen Verriegelungsbolzen;
- Zieldraht für die Schenkelhalsklinge (Durchmesser etwa 3,0 mm), mit zugehörigem Einschlaginstrument für die Schenkelhalsklinge, einem Schraubenzieher für die distalen Verriegelungsbolzen und für den der Schenkelhalsklinge zugeordneten Verriegelungsstift (Madenschraube).

Sämtliche in den Anmeldungsunterlagen offenbarten Merkmale werden als erfindungswesentlich beansprucht, soweit sie einzeln oder in Kombination gegenüber dem Stand der Technik neu sind.

## Patentansprüche

1. Osteosynthese-Hilfsmittel (10) zur Versorgung subtrochanterer, pertrochanterer und Schenkelhalsfrakturen mit einem von proximal in den Markraum eines Femur (11) einführbaren Verriegelungsnagel (12), der einen distalen Bereich (15) sowie einen proximalen Abschnitt (14) mit einer schrägen Durchgangsöffnung (16) aufweist, durch die hindurch von lateral in den Schenkenhals (17) und Femurkopf (18) ein Schenkelhalsteil (19) einführbar ist,
**dadurch gekennzeichnet**,
daß der Schenkelhalsteil (19) an seinem äußeren, dem Femurkopf (18) abgewandten Ende einen radial vorspringenden Absatz, insbesondere einen sich über den Umfang erstreckenden Kragen (33) aufweist, der die laterale Einführung des Schenkelshalsteils (19) in die schräge Durchgangsöffnung (16) des Verriegelungsnagels (12) begrenzt.

2. Hilfsmittel nach Anspruch 1,
**dadurch gekennzeichnet**,
daß zumindest der in Femurkopf (18) verankerbare Abschnitt (34) des Schenkelhalsteils, welcher vorzugsweise als Schenkelshalsklinge (19) ausgebildet ist, als Doppel-T bzw. I- (35), T-, Stern-, U- oder dgl. -Profil ausgebildet ist, wobei dieses vorzugsweise noch eine zentrale Duchgangsöffnung (36) für einen Führungsdraht umfaßt.

3. Hilfmittel nach Anspruch 2,
**dadurch gekennzeichnet**,
daß das vordere bzw. im Femurkopf (18) liegende Ende bzw. die vorderen Kanten des Profilabschnitts (34) des Schenkelhalsteils, insbesondere der Schenkelhalsklinge (19) als Schneide bzw. Schneiden (37) ausgebildet ist bzw. sind.
